# EUROPEAN PATENT APPLICATION

(11) **EP 1 637 180 A1**
(43) Date of publication of application: **22.03.2006**
(21) Application number: 05253920.2
(22) Date of filing: 23.06.2005
(51) Int. Cl.: A61N 1/372

(54) **Low Frequency transcutaneous telemetry to implanted medical device**

(30) Priority: 24.06.2004 US 876058
(71) Applicant: ETHICON ENDO-SURGERY, INC., Cincinnati, Ohio 45242 (US)
(72) Inventor: Hassler Jr, William L., Cincinnati Ohio 45249 (US); Dlugos Jr, Daniel F., Morrow Ohio 45152 (US)
(74) Representative: Tunstall, Christopher Stephen

(57) **Abstract**

An implantable medical device advantageously utilizes low frequency (e.g., 100 kHz or below) for telemetry communication with an external control module avoiding power dissipation through eddy currents in a metallic case of an implant and/or in human tissue, thereby enabling smaller implants using a metallic case such as titanium and/or allow telemetry signals of greater strength for implantation to a greater depth.

## Description

### Cross Reference to Related Applications

The present application is related to four co-pending and commonly-owned applications filed on even date herewith, the disclosure of each being hereby incorporated by reference in their entirety, entitled respectively:
"TRANSCUTANEOUS ENERGY TRANSFER PRIMARY COIL WITH A HIGH ASPECT FERRITE CORE" to James Giordano, Daniel F. Dlugos, Jr. & William L. Hassler, Jr., Serial No. ;
"MEDICAL IMPLANT HAVING CLOSED LOOP TRANSCUTANEOUS ENERGY TRANSFER (TET) POWER TRANSFER REGULATION CIRCUITRY" to William L. Hassler, Jr., Ed Bloom, Serial No. ;
"SPATIALLY DECOUPLED TWIN SECONDARY COILS FOR OPTIMIZING TRANSCUTANEOUS ENERGY TRANSFER (TET) POWER TRANSFER CHARACTERISTICS" to Resha H. Desai, William L. Hassler, Jr., Serial No. ; and
"LOW FREQUENCY TRANSCUTANEOUS ENERGY TRANSFER TO IMPLANTED MEDICAL DEVICE" to William L. Hassler, Jr., Daniel F. Dlugos, Jr., Serial No. .

### Field of the Invention

The present invention pertains to a telemetry system and, in particular, to a low frequency telemetry system that can be used in conjunction with a low frequency transcutaneous energy transfer (TET) system to transmit data between an external control module and a medical implant.

### Background of the Invention

It is known to surgically implant a medical device in a patient's body to achieve a number of beneficial results. In order to operate properly within the patient, a reliable, consistent communication link between the medical implant and an external control module is often necessary to monitor the implant's performance or certain patient parameters and/or to command certain operations by the implant. This communication link has traditionally been achieved with telemetry systems operating at frequencies from 100kHz. to upwards of 30MHz. These higher frequencies have been used to minimize the required coil size, thus enabling the coil to fit inside the implant case. It is also known to place a telemetry coil outside of an implant case in order to use a larger coil. Doing so, however, increases the complexity and expense of the implant since electrical leads must extend outside of the implant case to the coil, posing challenges to maintain a hermetic seal to the case and to avoid damage to the external coil.

While high frequency telemetry signals reduce the required coil size, such signals also reduce the effective communication distance between the transceivers in the system. Oftentimes, the implanted transceiver must be placed just under the surface of the patient's skin in order to effectively communicate with the external transceiver. At the shorter wavelengths (i.e., higher frequencies), the signals dissipate over a shorter distance when passing through tissue.

High frequency telemetry signals above 100kHz have a greater likelihood of electromagnetic interference or compatibility issues with other communication devices, and thus additional constraints arise under federal regulations. Conformance increases the time and complexity involved in developing the implant as well as limiting transmission power.

As an example of an implantable device that may benefit from use of telemetry is an artificial sphincter, in particular an adjustable gastric band that contains a hollow elastomeric balloon with fixed end points encircling a patient's stomach just inferior to the esophago-gastric junction. These balloons can expand and contract through the introduction of saline solution into the balloon. In generally known adjustable gastric bands, this saline solution must be injected into a subcutaneous port with a syringe needle to reach the port located below the skin surface. The port communicates hydraulically with the band via a catheter. While effective, it is desirable to avoid having to adjust the fluid volume with a syringe needle since an increased risk of infection may result, as well as inconvenience and discomfort to the patient.

Unlike the previously mentioned medical implants, an infuser device for an artificial sphincter is typically implanted below a thicker dermal layer of skin and adipose tissue. This is particularly true for patients that typically receive an adjustable gastric band as a treatment for morbid obesity. Moreover, being more deeply implanted may allow for greater client comfort. However, the thickness of tissue presents difficulties for effective communication.

Consequently, in order to provide for a larger effective communication range between the primary and secondary transceivers, and also to minimize the issue of FCC conformance, a significant need exists for enhancing telemetry with a deeply implanted medical device at a lower frequency than commonly used.

### Brief Summary of the Invention

The invention overcomes the above-noted and other deficiencies of the prior art by providing a telemetry system for an implantable medical device that operates at a frequency less than 100kHz, advantageously minimizes eddy current losses and allow uses of metallic cases to achieve smaller implant sizes. In instances where the telemetry carries significant power, the lower frequency avoids heating human tissue. Moreover, the low frequency telemetry system includes a telemetry coil encompassed within a hermetically sealed implantable device, ensuring the integrity of the device.

In one aspect of the invention, telemetry circuitry communicates across a physical boundary between primary and secondary resonant tank circuits having an inductance and capacitance combination selected for resonance within a range of 25 to 100 kHz. Thereby, an implantable medical device may be deeply implanted with an integral secondary telemetry coil yet achieve reliable telemetry.

These and other objects and advantages of the present invention shall be made apparent from the accompanying drawings and the description thereof.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIG. 1 is a block diagram illustrating a remote control system including low frequency power and telemetry systems of an implantable medical device system in accordance with the present invention;

FIG. 2 is a schematic diagram illustrating the low frequency TET power system and telemetry system of the present invention;

FIG. 3 is a more detailed schematic of an exemplary version of the telemetry transceiver including signal filtering circuitry;

FIG. 4a is a diagram illustrating magnetic fields between primary and secondary power and telemetry coils of the remote control system of FIG. 1; and

FIG. 4b is a diagram illustrating the magnetic fields between the primary and secondary coils of the power and telemetry systems of FIG. 1 for an alternative embodiment in which the primary power and telemetry coils are placed around a ferrite core.

### Detailed Description of the Invention

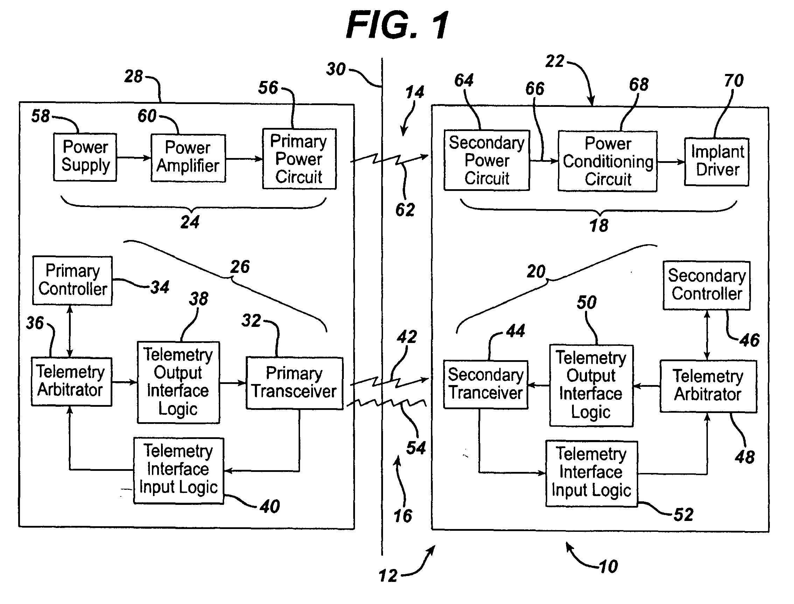

Referring now to the drawings in detail, wherein like numerals indicate the same elements throughout the views, in FIG. 1, a remotely controlled implantable medical device system 10 includes a remote control system 12 that advantageously performs both transcutaneous energy transfer (TET) through a TET power system 14 and telemetry through a Telemetry system 16. Internal portions 18, 20 of the TET power system 14 and the telemetry system 16 respectively reside in an implantable medical device ("implant") 22 and external portions 24, 26 of both respectively reside in an external control module 28. The implant 22 and external control module 28 are spaced apart by a physical boundary 30, which in the illustrative version is composed of dermal tissue typically including a thick layer of adipose tissue.

Implantable, bi-directional infusing devices that would benefit from enhanced TET powering and telemetry are disclosed in four co-pending and co-owned patent applications filed on May 28, 2004, the disclosure of which are hereby incorporated by reference in their entirety, entitled 1) "PIEZO ELECTRICALLY DRIVEN BELLOWS INFUSER FOR HYDRAULICALLY CONTROLLING AN ADJUSTABLE GASTRIC BAND" to William L. Hassler, Jr., Serial No. 10/857,762 ; (2) "METAL BELLOWS POSITION FEED BACK FOR HYDRAULIC CONTROL OF AN ADJUSTABLE GASTRIC BAND" to William L. Hassler, Jr., Daniel F. Dlugos, Jr., Rocco Crivelli, Serial No. 10/856,971 ; (3) "THERMODYNAMICALLY DRIVEN REVERSIBLE INFUSER PUMP FOR USE AS A REMOTELY CONTROLLED GASTRIC BAND" to William L. Hassler, Jr., Daniel F. Dlugos, Jr., Serial No. 10/857,315 ; and (4) "BI-DIRECTIONAL INFUSER PUMP WITH VOLUME BRAKING FOR HYDRAULICALLY CONTROLLING AN ADJUSTABLE GASTRIC BAND" to William L. Hassler, Jr., Daniel F. Dlugos, Jr., Serial No. 10/857,763 .

The external portion 26 of the telemetry system 16 includes a primary transceiver 32 for transmitting interrogation commands to and receiving response data from implant 22. Primary transceiver 32 is electrically connected to a primary controller 34 for inputting and receiving command data signals from a user or automated programmer. In particular, the primary controller 34 is in communication with a primary telemetry arbitrator 36 that is responsible for deconflicting and buffering downlink telemetry communication via primary telemetry output interface logic 38 to the primary transceiver 32 and uplink telemetry communication from the primary transceiver 32 via primary telemetry interface differential amplifier-based input logic 40. Primary transceiver 32 resonates at a selected radio frequency (RF) communication frequency to generate a downlink alternating magnetic field 42 that transmits command data to implant 22.

The internal portion 18 of the telemetry system 16 also includes a secondary transceiver 44 in a spaced relationship from primary transceiver 32 and is located on the opposite side of boundary 30 within the casing (not shown) of implant 22. In the present invention, secondary transceiver 38 is electrically connected to a secondary controller 46. In particular, the secondary controller 46 is in communication with a secondary telemetry arbitrator 48 that is responsible for deconflicting and buffering uplink telemetry communication via secondary telemetry output interface logic 50 to the secondary transceiver 44 and downlink telemetry communication from the secondary transceiver 44 via secondary telemetry interface differential amplifier-based input logic 52. Secondary transceiver 44 is magnetically coupled to primary transceiver 32 via alternating magnetic field 36 for downlink communication and via alternating magnetic field 54 for uplink communication. Magnetic flux from primary transceiver 32 generates an electrical command signal in secondary transceiver 44. The command signal is applied to a secondary controller 46 in implant 22 to direct operation of implant 22. Similarly, secondary transceiver 44 is electrically connected to controller 46 to transmit command response data from implant 22 to the external portion 26 of the telemetry system 16. When data transmission is requested, transceiver 44 resonates at the selected RF frequency to generate the uplink alternating magnetic field 54. Uplink magnetic field 54 is coupled into primary transceiver 32, which generates an electrical signal that is input to the primary controller 34.

Still referring to FIG. 1, the external portion 24 of the TET power system 14 also includes a primary power circuit 56 that is electrically coupled to a power supply 58 via a power amplifier 60 to resonate at a selected power signal RF frequency. An alternating magnetic field 62 is generated by primary circuit 56 in response to an electrical signal provided by power supply 58.The internal portion 18 of the TET power system 14 includes a secondary power circuit 64 in a spaced relationship from primary power circuit 56. Secondary power circuit 64 is located on the opposite side of boundary 30 from primary power circuit 56 within implant 22. Secondary power circuit 64 is electrically coupled to primary power circuit 56 via alternating magnetic field 62. Secondary power circuit 64 generates an electrical power signal 66 from magnetic field 62. Power signal 66 is rectified and regulated by a power conditioning circuit 68 and applied to an implant driver 70 to power various active components of the implant 22.

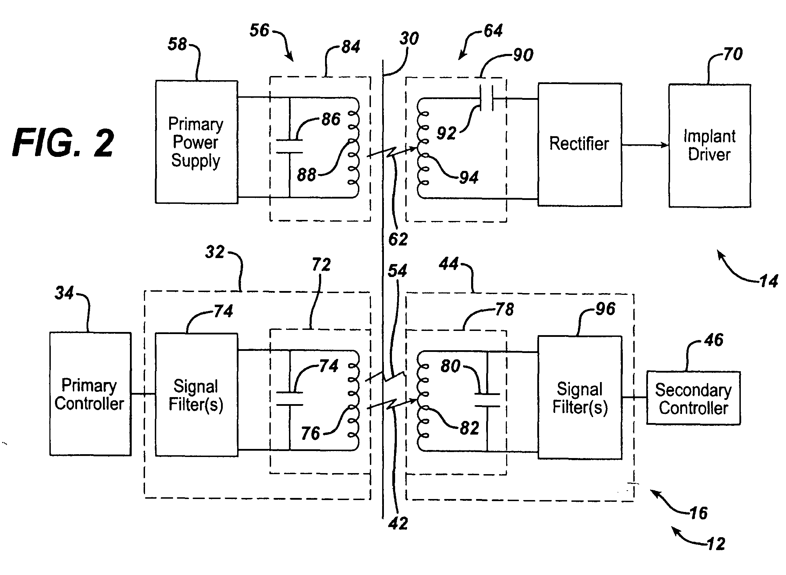

In FIG. 2, resonant circuitry portions are shown of the TET power system 14 and Telemetry system 16 of the remote control system 12. In particular, the primary transceiver 32 comprises a parallel tuned tank circuit 72 having a capacitance made up of one or more capacitors 74 connected in parallel with an inductive coil 76. Capacitance 74 and coil 76 are tuned to resonate at a particular frequency when a voltage is applied by controller 34. Similarly, secondary transceiver 44 comprises a parallel tuned tank circuit 78 having a capacitance 80 and inductive coil 62 tuned to resonate at the same frequency as primary telemetry tank circuit 72. Also as shown in FIG. 2, primary power circuit 56 comprises a parallel tuned tank circuit with a capacitance 86 and coil 66 tuned to a low power frequency. Secondary power circuit 64 comprises a series tuned tank circuit with a capacitance 92 and coil 94 that are also tuned to a low frequency level. In an illustrative version of the TET system, primary power circuit 56 transmits approximately one Watt of power at a resonant frequency under 10 kHz, and particularly under 5 kHz, by matching a high Q, low impedance primary tuned tank circuit 84 with a lower Q, low impedance secondary tuned tank circuit 90.

The TET power system 14 is described in further detail in the above-identified commonly assigned co-pending U.S. patent application Serial No. entitled ""LOW FREQUENCY TRANSCUTANEOUS ENERGY TRANSFER TO IMPLANTED MEDICAL DEVICE" filed on even date herewith and previously incorporated by reference. In the present invention, primary power circuit 56 operates at low frequency levels in order to effectively communicate with secondary power circuit 64 through the implant casing, as well as multiple layers of body tissue. For purposes of this discussion, the terms "low frequency" and "low frequency level" refer to frequencies below 100 kilohertz (kHz). As mentioned above, power coils 88, 94 also resonate at a low frequency to enable secondary power coil 94 to be encased within the sealed implant enclosure.

To transmit both power and telemetry magnetic fields 62, 42, 54 at low frequency levels, signal filter 96 filters the electrical signals received on the secondary transceiver 44, specifically from tank circuit 78. Filter 96 decouples the lower energy telemetry magnetic field 42 from the higher energy power field 64. Filters 96 may be any type of filter scheme selected to block frequencies other than the telemetry resonant frequency.

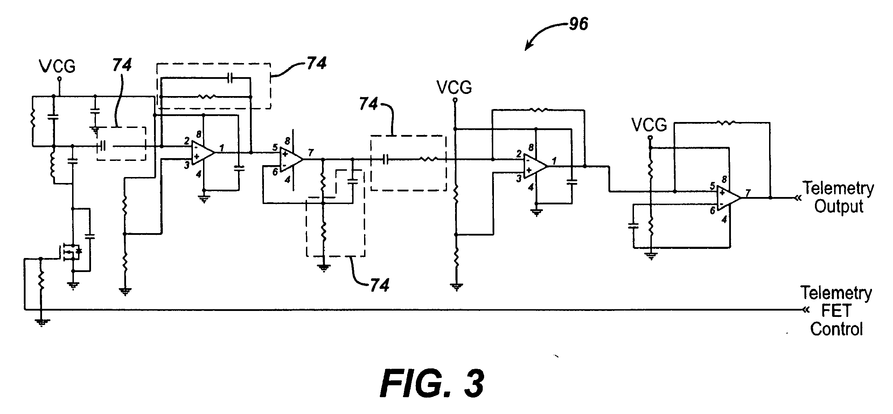

FIG. 3 illustrates one exemplary version of a filter 96 suitable for use in the present invention. In this version, the command signal from either the primary or secondary telemetry coil 82 is applied to a series of single pole low and high pass filter stages that isolate the telemetry signal from the TET power signal. For the single pole embodiment shown in FIG. 3, AC magnetic fields 62, 42, 54 are transmitted in alternate intervals to decouple the high Q of the power field 62 from the telemetry signals 42, 54. In another embodiment, filter 74 comprises one or more 2 pole filters such as, for example, a Chebyshev filter. The 2-pole filters provide more effective filtering of the high Q power signal, and enable AC magnetic fields 62, 42, 54 to be transmitted simultaneously. In order to effectively filter the lower energy telemetry signal from the higher energy power signal, the resonant frequencies of the two signals are separated by at least one decade of frequency.

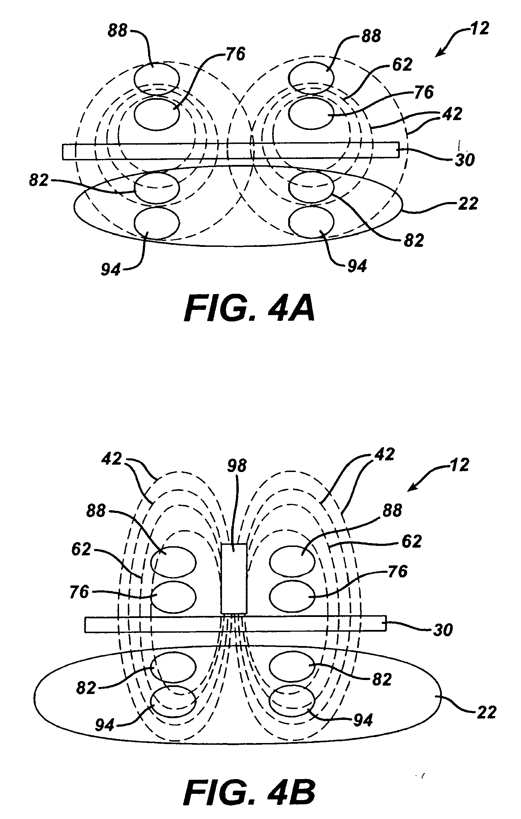

FIG. 4A and 4B illustrate magnetic fields 62 and 42/54 respectively radiating from primary power coil 88 and primary transceiver coil 76 to subcutaneous secondary TET coil 94 and telemetry coil 82. In the version illustrated in FIG. 4A, magnetic fields 62 and 42/54 both have a double circular toroidal shape that only penetrates in a shallow manner cross physical boundary 30 to respective secondary TET power and telemetry coils 94, 82, thereby reducing the energy transfer between the coils and necessitating corresponding shallow placement of the implant device 22. FIG. 4B illustrates an alternative embodiment for the invention, described in greater detail in the previously referenced patent application entitled "TRANSCUTANEOUS ENERGY TRANSFER PRIMARY COIL WITH A HIGH ASPECT FERRITE CORE", in which the primary power and transceiver coils 56, 66 are placed around a magnetically conductive ferrite core 98. As shown in FIG. 4b, the addition of ferrite core 98 causes the magnetic flux 62, 42/54 from primary coils 88, 76 to be drawn towards the core 98. Magnetic fields 62, 42/54 thus collapse radially into core 98 and change from a circular shape to an elliptical shape. The elliptical shape of fields 62, 42/54 increases the coupling efficiency between both the primary and secondary telemetry coils 76, 82 and the primary and secondary power coils 88, 94. The increased coupling efficiency with ferrite core 98 provides improved telemetry between transceivers 32, 44 at increased physical distances or at a lower power level.

In an experimental embodiment of the present invention, primary and secondary transceiver coils 76, 82 were each formed of 220 turns of 36 gauge magnet wire. Coils 76, 82 were each placed in parallel with a capacitance that resulted in a resonant frequency for the tank circuit of approximately 25 kHz. The primary power coil 88 was formed of 102 turns of litz wire made up of 100 individually insulated 30-gauge magnet wire. The magnet wires were connected in parallel with 9.2 microfarads of capacitance, which created a parallel tuned tank circuit with a high Q and a resonant frequency under 10 kHz, and particularly under 5 kHz. Both the primary power coil 88 and primary telemetry coil 76 were placed around a ferrite core 98 having a length of 3 inches and a diameter of 0.75 inches. With these parameters and resonant frequencies, the primary coil 88 transmitted approximately one watt of power and the primary telemetry coil 76 transmitted power in the milliwatt range. The power and telemetry coils 88, 76 alternated transmission intervals, with the telemetry system 16 transmitting data at a baud rate of 1 kHz. In this experimental embodiment, a distance of 3 inches separated the primary and secondary coils.

In designing a low frequency telemetry system for a deeply implanted medical device, it is desirous to make the Qs of the two magnetically coupled telemetry coils in their parallel tuned tank circuit to be within a range of 10 to 20. If the Qs of the two tank circuits are below this range, it will be difficult to achieve any significant deep penetration telemetry range. If the Qs were above this range, it would be difficult to manufacture the system in high quantities without individually tuning each pair of parallel tuned tank circuits.

It is also possible to have the primary (or external) telemetry tank circuit be of very Q (greater than 100) while having a lower Q (around 10) in the implant. An advantage of doing this as opposed to having the high Q circuit in the implant is that a higher Q usually requires a larger and heavier coil, and inductance. This arrangement would still allow for the natural frequency of the high Q circuit to fall within the effective frequency range of the low Q circuit without the need for individual circuit tuning or matching.

The coils in the deep implantation telemetry system may have their number of coil turns maximized to couple better with and better generate the AC magnetic field that is the telemetry medium. This needs to be done without creating a significantly high impedance at resonance in the parallel tuned tank circuits. The open cross sectional area within the perimeter of the coil also needs to be maximized in order to improve the magnetic coupling between the tank circuits. The coils used had 220 turns of 36-gauge magnet wire which when put in parallel with 5600 pF of capacitance, created a resonant frequency of 25 kHz, with a calculated Q of 19, and a calculated impedance of around 20 kilo-Ohms at resonance. The actual Q is always around 10% to 30 % lower than the calculated value due to parasitic losses, and other non-linear effects.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, while the telemetry system 16 described has particular advantages for an implantable medical device system 10, aspects consistent with the present invention have application to other scientific and engineering scenarios including inanimate physical boundaries. For instance, in a processing apparatus it may be desirable to monitor and/or control an actuator that is contained within a vessel without compromising the integrity of the vessel with wires or conduits passing therethrough.

Furthermore, telemetry system 16 may be used in the absence of a TET power system 14. As yet another alternative, telemetry system 16 may provide a one-way communication channel rather than a two-way channel.

## Claims

1. A remote control system, comprising:
a primary controller;
a primary telemetry transmitter energized by the primary controller and including a primary coil in electrical communication with capacitance to form a resonant tank circuit having peak resonance up to 100 kHz;
a secondary controller; and
a secondary telemetry receiver communicating received electromagnetic energy transferred from the primary telemetry transmitter to the secondary controller and including a secondary coil in electrical communication with capacitance to form a resonant tank circuit having peak resonance up to 100 kHz.

2. The remote control system of claim 1, wherein the peak resonance of the resonant tank circuits of the primary telemetry transmitter and secondary telemetry receiver is between 25 and 100 kHz.

3. The remote control system of claim 1, further comprising a medical implant housing encompassing the secondary controller and the secondary telemetry receiver.

4. The remote control system of claim 1, wherein the coil further comprises a longitudinally aligned ferrite core.

5. The remote control system of claim 1, wherein the primary coil comprises multi-turn insulated Litz wire.

6. The remote control system of claim 1, wherein the secondary coil comprises multi-turn Litz wire.

7. The remote control system of claim 1, wherein the primary controller and primary telemetry transmitter reside external to a physical boundary spacing apart the secondary controller and secondary telemetry receiver.

8. The remote control system of claim 6, wherein the primary telemetry transmitter comprises a primary transceiver and the secondary telemetry receiver comprises a secondary transceiver for two-way telemetry and control.

9. The remote control system of claim 1, further comprising bandpass filtering between the secondary telemetry receiver and the secondary controller.

10. An implantable medical device system, comprising:
an external control module, comprising:
a primary telemetry coil having a resonant frequency up to 100 kHz, and
a primary controller in electrical communication with the primary telemetry coil ; and
an implantable medical device, comprising:
an enclosure,
a secondary telemetry coil having a resonant frequency up to 100 kHz, and
a secondary controller in electrical communication with the secondary telemetry coil.

11. An implantable medical device responsive to an external primary telemetry coil inductively coupling a telemetry signal having a resonant frequency of up to 100 kHz, the implantable medical device comprising:
an enclosure,
a secondary telemetry coil having a resonant frequency of up to 100 kHz, and
a secondary controller in electrical communication with the secondary TET telemetry coil.
